# EUROPEAN PATENT SPECIFICATION

(11) **EP 4 186 558 B1**
(45) Date of publication and mention of the grant of the patent: **08.01.2025**
(21) Application number: 21383068.0
(22) Date of filing: 25.11.2021
(51) Int. Cl.: A61N 1/04, A61N 1/36

(54) **ELECTRICAL STIMULATION ELECTRODES AND ELECTRICAL STIMULATION OF A PERSON**
ELEKTRODEN ZUR ELEKTRISCHEN STIMULATION UND ELEKTRISCHE STIMULATION EINER PERSON
ÉLECTRODES DE STIMULATION ÉLECTRIQUE ET STIMULATION ÉLECTRIQUE D'UNE PERSONNE

(43) Date of publication of application: 31.05.2023
(73) Proprietor: Fundación Tecnalia Research & Innovation, 20009 San Sebastián, Guipúzcoa (ES)
(72) Inventor: Keller, Thierry, San Sebastián (ES); Imaz Ojanguren, Eukene, San Sebastián (ES); Bijelic, Goran, San Sebastián (ES); Idigoras Leibar, Igone, San Sebastián (ES)
(74) Representative: Balder IP Law, S.L.

(56) References cited:
- WO-A1-00/65993
- WO-A1-2006/089319
- CA-A- 1 219 642
- DE-A1- 4 231 236
- US-A- 5 114 424
- US-A1- 2010 152 794
- US-B2- 10 118 035
- US-B2- 11 071 192

## Description

### TECHNICAL FIELD

The present disclosure relates to the field of electrical stimulation of parts of a body of a person. More particularly, the present disclosure relates to stimulation electrodes, devices for applying electrical stimulation through the skin of a person, and methods associated therewith.

### STATE OF THE ART

Electrical stimulation of muscles and nerves is sometimes applied for rehabilitation or to figure out a medical condition of a person suffering a disease or injury. For example, Transcutaneous Electrical Nerve Stimulation (TENS) is widely used for alleviating pain, while Current Perception Threshold (CPT) is used to support the evaluation of peripheral sensory nerves.

A stimulating apparatus is an apparatus configured to generate stimulation signals that, once coupled to the parts of the body of the person, produce stimuli thereon. For this to occur, a device must be arranged between the stimulating apparatus and the body of the person.. A non-invasive stimulation electrode transfers an electric current to the external skin of the person.

For the stimulation to be effective, the stimuli that is applied on the person in a certain way. This means that the electric currents must not only feature certain frequency but also travel on the part of the body in a certain manner. Accordingly, for the electric current to travel along the body of a person at least two electrodes are required. The arrangement of the conducting electrodes, thus influences the stimuli applied to the person, and if the design is not carefully planned the stimuli may have an adverse effect on the person, including but not limited to burn the person due to a high current density. The human skin is inhomogeneous by nature, therefore, the current fields applied through the skin by means of two electrodes placed far apart travel through the body in an unknown manner, always taking the path with least impedance. For some applications like the CPT or TENS it might be important to delimit the area where the current fields will be applied. Delimiting the stimulation area will prevent the electrical fields to reach other body parts and prevent undesired or ineffective results.

The electrodes shall also be designed so as to reduce the possibility of being detached from the skin of the person, which is typically curved, soft and is susceptible to movement.

Commercially available hydrogel electrodes are multi-layered. These electrodes usually consist of a hydrogel layer, which is to be in contact with the skin and provides the interface for transferring the electrical fields to it; an electrically conductive layer, which consists of a mesh made of any conductive material such as copper; and a dielectric layer which is usually made of fabric and is glued to and covers the conductive layer. Commercial hydrogel electrodes are single electrodes and thus, they usually include either a snap or pin connector, which is attached to the conductive layer by means of an internal cable, which is trapped between the conductive layer and the dielectric layer. These electrodes are available in a variety of solid shapes such as oval, circle, square or rectangular and different sizes, and in order to stimulate a person at least two electrodes are placed on top of the skin of the person separated with a minimum distance. In most cases, corners are rounded to avoid the "edge effect", which consist of high current densities that take place in sharp corners. The electrical fields generated between these conventional electrodes in the body of the person are not delimited, as current travels longitudinally from one electrode to the other electrode, but it can be spread over other surrounding areas of the body of the person. The manufacturing process of these electrodes is complex and has many limitations, which does not make it suitable for the manufacturing of multi-field electrodes.

Screen-printing is a technique that together with conductive inks have brought the possibility of manufacturing multi-field electrodes in a variety of shapes and sizes. However, some design limitations can arise as the different layers are printed one on top of the other in a 2D format.

When the conductive areas feature simple designs the conductive tracks can be all arranged in the same layer without any crossing between them. However, there might be more complex multi-field electrode designs where tracks may need to overlap, causing a short-circuit, and therefore, becoming non-functional. One solution for the manufacturing of such electrodes might be the use of several layers, alternating conductive and dielectric layers. However, due to manufacturing tolerances, adding layers involves increasing the risk of short-circuits and open-circuits. Multi-layering also makes the electrodes less flexible and less adaptable to different body shapes, and thus, increases the risk of burns due to bad electrode-skin attachment. When tracks may need to overlap stimulation fields, another solution would be to leave space for the track, removing conductive ink from the stimulation field, or leaving an opening. However, this might increase the risk of presenting an "edge effect" and burning the skin, as the stimulation fields may turn to have sharp corners or reduced areas producing high current densities.

There is an interest in providing both a stimulation electrode and a multi-field electrode that can be manufactured in a cost-effective manner, that ensures that the electrical fields travel through a delimited area of the body and that reduces the risk of burns. Likewise, there is an interest in providing a stimulating apparatus that uses these electrodes for electrical quantitative sensory assessment.

US11071192B2 discloses a flat flexible support piece for a dielectrically impeded plasma treatment. US10118035B2 discloses systems and methods for enabling appetite modulation and/or improving dietary compliance using an electro-dermal patch. US2010/152794A1 discloses an apparatus for providing nerve stimulation and related methods. WO2006/089319A1 discloses a planar electrode. WO00/65993A1 discloses a medical electrode. CA1219642A discloses a multi-element electrosurgical indifferent electrode with temperature balancing resistors. DE4231236A1 discloses a flat electrode for highfrequency surgery. US5114424A discloses a multipart planar electrode for an HF-surgery device.

### DESCRIPTION OF THE INVENTION

The invention is defined in the appended claims. Aspects, embodiments and examples disclosed herein which do not fall within the scope of the appended claims do not form part of the invention, and are merely provided for illustrative purposes.

A first aspect of the disclosure relates to a stimulation electrode comprising: a dielectric substrate; an electrically conductive layer on the dielectric substrate comprising: first and second electrically conductive areas, first and second electrically conductive tracks connected with the first and second areas, respectively, the second area enclosing the first area, and the second area having an opening that forms a passageway through which the first track passes; a dielectric layer at least covering the conductive tracks; an adhesive layer at least on a portion of the dielectric layer; and an electrically conductive hydrogel layer at least covering the first and second areas and the opening.

Electric current supplied to the stimulation electrode can travel from the first area to the second area or vice versa through the skin of the person. Since the first area is enclosed within the second area, the currents travel within the surface delimited by the second area, thereby resulting in a controlled localization of the applied electrical stimuli on the body of the person.

Despite the second area surrounds the first area (and, thus, does not contact it) and the conductive tracks are in the same layer as the first and second areas, the opening provides the passageway for the conductive track of the first area without requiring additional conductive layers nor any special connecting means. Accordingly, a first end of the first track is connected with the first area and a second end of the first track can be connected with a stimulating apparatus (for example, the apparatus of the fourth aspect below) even though the connecting point with said apparatus is outside of the surface delimited by the second electrically conductive area, so the first track extends from an inner part of the second area to an outer part thereof. This means that electrical connection even with intricate conductive area designs is possible in this fashion, which in turn simplifies the manufacturing process of the electrode and makes its operation more reliable because the electrical connections are less prone to breaking when the electrode is attached to the body of the person.

The hydrogel layer at least covering the second area and the opening diminishes possible edge effects, especially those that may be produced at ends of the second area that form the opening. The dielectric layer covers the track that goes through the opening, so no current leakages will go through the hydrogel layer. Besides, even if there is no conductive area underneath the hydrogel in the opening area, keeping a continuous layer of hydrogel, without opening, will prevent high current densities happening in the edges of the opening, allowing the current to flow in a more homogeneous manner. Hence, the density of currents is reduced in this location and the risk of burning the person is likewise reduced.

The areas covered by the hydrogel remain free from the adhesive layer, in this way electrically conductive areas, are in contact with the skin of the person and the stimuli can be applied using the entire conductive surface.

Preferably, the electrically conductive layer comprises biocompatible electrically conductive ink, and the adhesive layer comprises biocompatible adhesive. The hydrogel layer is also preferably biocompatible. By way of example only, the ink may be silver-based ink (e.g. silver chloride ink like CI-4040 for medical use, with e.g. 80% of silver and 20% chloride), and the hydrogel layer may be AG730. It will be noted that other inks and layers are also possible within the scope of the present disclosure.

In some embodiments, the first and second areas are concentric.

In some embodiments, the first area and the second area have a perimeter with a same shape except for the opening that the second area defines.

Concentric areas make a more reproducible distribution of the electric currents on the skin possible because the electric currents travel from/to the first conductive area to/from the second conductive area and the relative distance between them is predefined by electrode design and thus, not influenced by the user.

The shapes of the perimeters can be regular (e.g. polygons) or irregular.

In some other embodiments, the perimeters have a different shape (e.g. the perimeter of the first area is a circle, and the perimeter of the second area is a square).

The presence of alternate perimeters influences how the stimuli are applied to the body of the person. Sometimes, irregular shapes are convenient for covering certain parts of the body.

In some embodiments, the second area has an open geometry having two ends, the opening being formed in a space between the two ends. In some embodiments, the second area is C- or U- shaped.

In some embodiments, the two ends of the open geometry are apart by a distance equal to or less than 15% of a length of a longest side of a rectangular polygon circumscribing the second area, namely each side of the rectangular polygon being tangential to at least one point of the second area.

A narrow passageway is sufficient to let the first track pass from the inner part to the outer part whilst maintaining substantially even distribution of currents between the first electrically conductive area and the second electrically conductive area.

In some embodiments, the second area encloses the first area with an angle equal to or greater than 270°, preferably equal to or greater than 300° and more preferably equal to or greater than 330°.

Having an enclosure as large as possible is convenient for stimulation of greater portions of the body of the person. However, spacing between the first track and the edges of the second conductive areas is preferably 0,5 mm or more (e.g. 1,0 mm, 1,5 mm, etc.) for avoidance of manufacturing errors due to manufacturing tolerances.

The angle is measured from a geometrical center of the first area and covers the entire portion that radially leads, from said geometrical center, to the second area.

In some embodiments, the electrode further comprises an electrically conductive hydrogel layer that at least covers the first and second electrically conductive areas.

The hydrogel layer makes the coupling of currents from the conductive areas to the body and vice versa more reliable and safer. Preferably, the hydrogel layer is applied to the second area and the opening in one application pass so that there is a continuum of the electrical fields between the hydrogel layer on the second area and the hydrogel layer on the opening.

A second aspect of the present disclosure relates to a sheet (namely a multi-field electrode) comprising a plurality of stimulation electrodes according to the first aspect of the disclosure such that the stimulation electrodes share the dielectric substrate.

The multi-field electrode is manufacturable in a cost-effective manner owing to the simplicity of the stimulation electrodes it comprises. The multi-field electrode also has reduced probability of burning the person during use thereof, and/or of breaks in the electrically conductive tracks that would render the electrode inoperable.

In some embodiments, the sheet comprises attaching means for attachment of the sheet to an apparatus for stimulation.

The attaching means may be, for example, holes in the substrate of the electrodes adapted to match protrusions in the apparatus. The attaching means are preferably arranged in a poka-yoke fashion such that they only allow one possible way of attachment to the apparatus for stimulation, thereby also simplifying the electrical connection between free ends of the conductive tracks and terminals of the apparatus.

Other attaching means are likewise possible, for example, screws, adhesive, Velcro, etc.

A third aspect of the present disclosure relates to a kit of stimulation electrodes, the kit comprising: N sheets (multi-field electrodes) according to the second aspect of the disclosure, where N is a natural number equal to or greater than 2; each sheet comprises an area with a plurality of free ends of the electrically conductive tracks of the stimulation electrodes; where for the second to the Nth sheet, and being M each such sheet, the dielectric substrate of the Mth sheet has a cutout that matches the area with the plurality of free ends of sheets preceding the Mth sheet.

Electrical stimulation procedures usually involve the stimulation of different or extensive parts of the body, for example different muscles, or the thigh which has a large volume. Although multi-field electrodes are flexible enough to be arranged on the body of the person, the use of a single multi-field electrode covering large portions of the body is not convenient because they the different portions of the sheet may detach from the body when the person slightly moves some body parts.

The provision of multiple sheets hence may improve the usability of the devices necessary for electrical stimulation. However, each sheet includes free ends of conductive tracks that have to be connected with terminals of the stimulating apparatus, and the terminals are typically arranged one next to another within the stimulating apparatus. Having a plurality of electrically conductive tracks terminating in free ends may result in big connector sites due to a big number of tracks and the manufacturing tolerances that require a minimum distance between tracks and/or free ends.

The kit has a plurality of multi-field electrodes that can be electrically connected with a stimulating apparatus in a simple manner in spite of the tightness with which the terminals are arranged on the stimulating apparatus. To this end, some sheets may have cutouts formed on the respective dielectric substrate so as to allow the connection of the free ends of conductive tracks of other sheets, particularly all the sheets e.g. underneath, with the terminals in the apparatus; so the N sheets can overlap without interfering in the free ends that shall be in contact with the stimulating apparatus. Said free ends are arranged such that they are present in the cutout portions of different sheets.

When all the sheets have a connection portion thereof arranged on the stimulating apparatus with the appropriate order, the different sheets leave one or more windows for connection of the free ends of the conductive tracks of sheets arranged the farthest away from the set of terminals of the apparatus.

A fourth aspect of the present disclosure relates to an apparatus for electrical stimulation of a person, comprising: at least one source for generating electrical stimulation signals for stimulation of the person; a set of connection terminals for electrical connection of stimulation electrodes; and one or more sheets (multi-field electrodes) according to the second aspect of the present disclosure, or a kit of stimulation electrodes according to the third aspect of the disclosure, each stimulation electrode thereof being electrically connected with one or more connection terminals of the set of connection terminals.

In some embodiments, the apparatus further comprises user input means, and at least one processor configured to sequentially select a frequency and an intensity of the stimulation signal, in each terminal of the set of connection terminals, according to a stimulation technique, the stimulation technique being at least based on user feedback (e.g. user input and/or non-input) to stimuli applied to the person.

In some embodiments, the at least one processor is further configured to sequentially select one, some or all electrodes among all stimulation electrodes to apply the stimulation signals.

In some embodiments, the apparatus further comprises attaching means for attachment of the apparatus to the one or more sheets or the kit.

The attaching means of the apparatus cooperates with attaching means of each sheet so as to allow simple attachment of the two and leading to an automatic electrical connection of free ends of conductive tracks to terminals of the set of terminals.

A fifth aspect of the present disclosure relates to a method of manufacturing a stimulation electrode, comprising: arranging or manufacturing a dielectric substrate; printing on the dielectric substrate, with electrically conductive ink, first and second electrically conductive areas, and first and second electrically conductive tracks connected with the first and second areas, respectively; adding a dielectric layer at least covering the conductive tracks; applying an adhesive layer to the dielectric layer; and applying an electrically conductive hydrogel layer to at least the first and second areas and the opening; the second area being printed such that it encloses the first area printed; and the second area being printed such that it defines an opening forming a passageway through which the first track passes.

The manufactured stimulation electrode comprises few layers, including a single electrically conductive layer with electrically conductive ink. The production of such stimulation electrodes is cost-effective and even allows the incorporation of complex conductive area designs.

In some embodiments, the first area and the second area are printed such that they have a perimeter with a same shape except for the opening that the second area defines. In some other embodiments, the perimeters have a different shape.

In some embodiments, the first and second areas are printed such that they are concentric.

In some embodiments, the second area is printed such that it has an open geometry having two ends, the opening being formed in a space between the two ends. In some embodiments, the second area is printed such that it is C- or U- shaped. In some embodiments, the ends of the open geometry are apart by a distance equal to or less than 15% of a length of a longest side of a rectangular polygon circumscribing the second area.

In some embodiments the second area is printed such that it encloses the first area with an angle equal to or greater than 270° and/or 300° and/or 330°.

In some embodiments, the electrically conductive hydrogel layer is applied covering the electrically conductive layer.

In some embodiments, the electrically conductive hydrogel layer is applied in a single deposition of electrically conductive hydrogel.

In some embodiments, the stimulation electrode is a stimulation electrode according to the first aspect of the present disclosure.

In some embodiments, the method is a method of manufacturing a sheet according to the second aspect of the disclosure or a kit according to the third aspect of the disclosure. In this sense, multiple stimulation electrodes are provided on a single dielectric substrate.

A sixth aspect of the present disclosure relates to a method of using the apparatus of the fourth aspect of the present disclosure for defining the electrical sensory thresholds of a person, also referred to as Electrical Quantitative Sensory Assessment (EQSA) in the present disclosure, comprises: attaching each sheet of stimulation electrodes on a part of a body of a person such that the hydrogel layer contacts a skin of the person; electrically connecting free ends of the tracks of each electrode to terminals of the set of connection terminals; applying stimuli at predetermined locations, and predetermined frequency and intensity of the stimulation signals after the attaching and electrically connecting steps; after the applying stimuli step, digitally selecting, based on each of user feedback (e.g. user input and/or non-input) to stimuli already applied to the person, the location, frequency and intensity of the stimulation signals, and applying further stimuli with the selected parameters; after the applying stimuli and applying further stimuli steps, digitally providing a digital file with data indicative of electrical sensory thresholds of the person based on both the applied stimuli parameters and the user feedback. The sixth aspect of the present disclosure is not according to the claimed invention.

The EQSA makes possible to perform an estimated analysis of the status of different afferent nerves of a person in one or more parts of her/his body, particularly due to sensory nerve problems.

As electrical stimuli are applied to the person, the person is able to give feedback to the apparatus intentionally or unintentionally via the user input means (e.g. a joystick, a touchscreen, a keyboard, one or more buttons, a pupil detector performing pupillometry, a sweat sensor, a heart rate sensor, etc.). The apparatus, in turn, reacts to the indications or lack of indications to sequentially adjust the stimuli to be subsequently applied to the person so as to find the location, frequency and intensity combinations that trigger some response from the person.

Based on the different stimuli applied and the response by the person, the sensory thresholds are estimated and registered in the digital file for ulterior analysis or revision by a physician, for example. This data could be represented in form of graphs to analyze the magnitude of the results or in form of body maps, where the sensory thresholds of the body of the person are visualized in a qualitative spatial representation manner.

In some embodiments, the apparatus further comprises attaching means for attachment of the apparatus to the one or more sheets or the kit; each sheet of the one or more sheets or the kit comprises attaching means for attachment of the respective sheet to the apparatus; the attaching means of the apparatus and the attaching means of each sheet are arranged to cooperate and automatically electrically connect free ends of the electrically conductive tracks with terminals of the set of connection terminals; and the electrically connecting step comprises attaching each sheet to the apparatus by way of respective attaching means such that the free ends become automatically electrically connected with the terminals.

The attachment and electrical connection of the multi-field electrodes is simplified, thereby resulting in a reliable attachment of the multi-field electrodes to the apparatus, and both quick and correct electrical connection of the conductive tracks with the terminals so as to avoid malfunction of the apparatus or inadequate application of stimuli, that is to say, avoid that the intended stimuli are not applied to the person or applied through a stimulation electrode not intended to apply a given stimulus.

### BRIEF DESCRIPTION OF THE DRAWINGS

To complete the description and in order to provide for a better understanding of the invention, a set of drawings is provided. Said drawings form an integral part of the description and illustrate embodiments of the invention, which should not be interpreted as restricting the scope of the invention, but just as examples of how the invention can be carried out. The drawings comprise the following figures:
Figures 1A to 1D show a stimulation electrode in accordance with embodiments.
Figure 2 shows a stimulation electrode in accordance with embodiments.
Figures 3A-3E show different layers of a stimulation electrode in accordance with embodiments.
Figures 4 to 6 partially show multi-field electrodes in accordance with embodiments.
Figure 7 partially shows a kit with of stimulation electrodes in accordance with embodiments.
Figure 8 shows a stimulation electrode or a multi-field electrode in accordance with embodiments.

### DESCRIPTION OF WAYS OF CARRYING OUT EMBODIMENTS

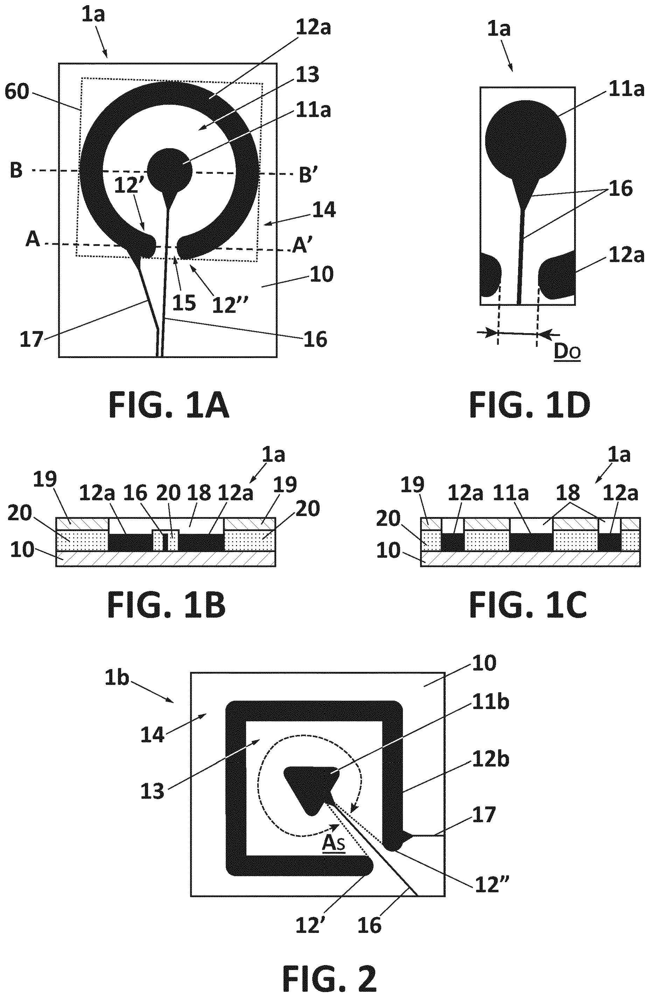

Figure 1A shows a stimulation electrode 1a in accordance with embodiments, Figure 1B and 1C show, in an exaggerated manner for clarity purposes only, the cross-sections taken at lines A-A' and B-B', respectively, and Figure 1 D shows a portion of the electrode 1a zoomed-in to illustrate a distance.

The electrode 1a includes a dielectric substrate 10, i.e. a layer made of dielectric material, that is preferably elastically deformable and flexible to at least follow the curvature of body parts of a person. By way of example, the dielectric substrate 10 may be of polyester (e.g. Autostat CT3), polyethylene terephthalate, namely PET, thermoplastic polyurethane, namely TPU, etc. For example, dielectric substrates 10 of polyester, PET or TPU having a thickness between 50 and 150 µm have an adequate balance between flexibility to follow the curvature of body parts and both rigidity and strength to support other layers.

The electrode 1a also includes an electrically conductive layer with first electrically conductive area 11a, a second electrically conductive area 12a, a first electrically conductive track 16 connected with the first area 11a and a second electrically conductive track 17 connected with the second area 12a.

The electrode 1a further includes an electrically conductive hydrogel layer 18, an adhesive layer 19, and a dielectric layer 20.

The second area 12a defines an enclosed surface 13 and a non-enclosed surface 14; the second area 12a also defines an opening 15 between two ends 12', 12" thereof. The first area 11a is within the enclosed surface 13, thereby being surrounded by the second area 12a.

The first track 16 can reach the non-enclosed surface 14 for connection with a stimulating apparatus via the opening 15. The second track 17 can likewise reach the stimulating apparatus since it can readily be outputted to the non-enclosed surface 14, although it could be outputted to the enclosed surface 13 and then exit it through the opening 15 as well if the designs of the conductive areas 11a, 12a so requires.

The opening 15 is preferably narrow to make the second area 12a to surround the first area 11a as much as possible. In this sense, the ends 12', 12" of the second 12a are preferably apart by a distance D_{O} that is equal to or less than 15% of a length of a longest side of a rectangular polygon 60 (shown with dashed lines for illustrative purposes only) circumscribing the second area 12a. As in these embodiments the perimeter of the second area 12a is circumferential, the rectangular polygon 50 is squared and thus all the four sides thereof have a same length, so Do is equal to or less than the length of such side. The distance Do is preferably greater than or equal to 0,5 mm, and is preferably smaller than or equal to 5,0 mm.

How much the second area 12a surrounds the first area 11a is shown in the embodiments of Figure 2 by way of angle A_{S}, which is measured between lines (shown in Figure 2 with dotted lines for illustrative purposes only) defined from the geometrical center of the first area to the ends of the second area and that radially lead from the first area to the second area.

For the sake of clarity only, in Figures 1B and 1C, the dielectric substrate 10 has been illustrated with a first stripes pattern, the adhesive layer 19 has been illustrated with a second stripes pattern, and the electrically conductive layer 12a, 16 has been illustrated with dark color.

In some embodiments, like the ones of Figures 1A-1D, the first and second areas 11a, 12a have the hydrogel layer 18, which also extends on the opening 15. The opening 15, including the first track 16, is covered with the dielectric layer 20, and the hydrogel layer 18 is on top of the dielectric layer 20. The enclosed and non-enclosed surfaces 13, 14 may have an adhesive layer 19.

Figure 2 shows a stimulation electrode 1b in accordance with embodiments.

The stimulation electrode 1b includes the same elements of electrode 1a as described with reference to Figures 1A-1D, but the first and second areas have different perimeter shapes. Unlike electrode 1a in which the first and second areas 11a, 12a both have circumferential perimeters, the first and second areas 11b, 12b of electrode 1b have triangular and square perimeters, respectively. In other embodiments, the first and second areas have other perimeter shapes, which can be shared or not.

As aforesaid, Figure 2 illustrates the level of surrounding of the second area 12b with respect to the first area 11b by way of angle A_{S}, which in this case is approximately 350°. The level of surrounding can be computed as the ratio of A_{S} divided by 360°; in this case, said level is 0,97, namely 97%.

In the embodiments of Figure 2, the opening 15 is formed on a corner of the second area 12b, but in other embodiments it is formed at a different location along one of the edges of the second area 12b; it can be at the middle point of the edge, or between a corner and the middle point. The formation of the opening 15 is preferable because it causes the second area 12b to have fewer corners in total, which tend to produce high density of currents thereon. Accordingly, in these embodiments, the second area 12b has three corners, whereas in embodiments in which the opening 15 is along one of the edges the second area would have four corners.

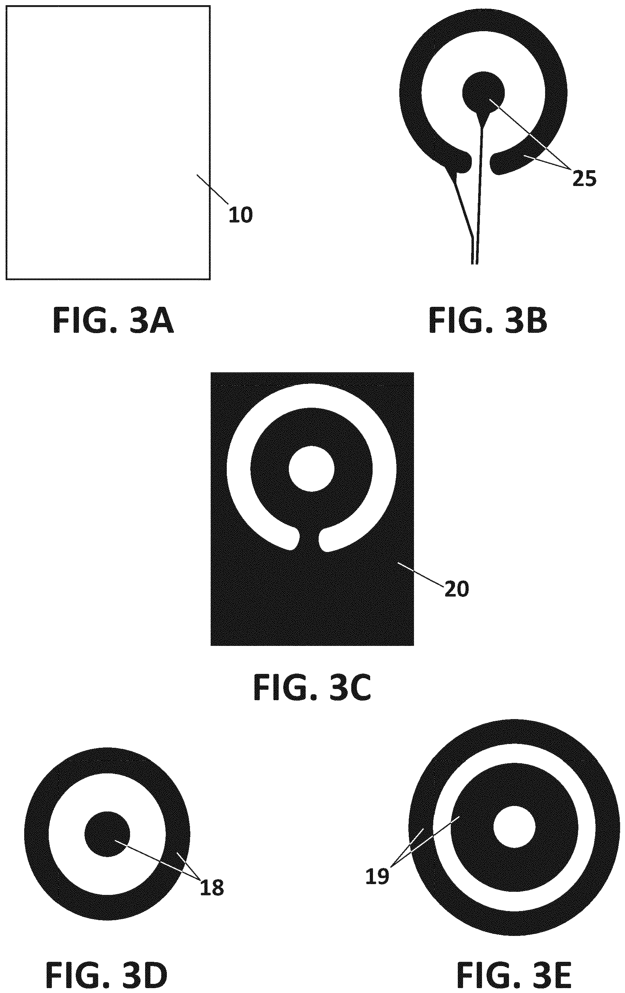

Figures 3A-3E show different layers of a stimulation electrode in accordance with embodiments. In this example, the layers correspond to a stimulation electrode as shown in Figures 1A-1D; it will be noted that other stimulation electrodes have the layers with other shapes.

Particularly, Figure 3A shows a dielectric substrate 10; Figure 3B shows an electrically conductive layer 25 with electrically conductive ink that provides the first and second electrically conductive areas and the first and second electrically conductive tracks; Figure 3C shows a dielectric layer 20; Figure 3D shows an electrically conductive hydrogel layer 18; and Figure 3E shows an adhesive layer 19.

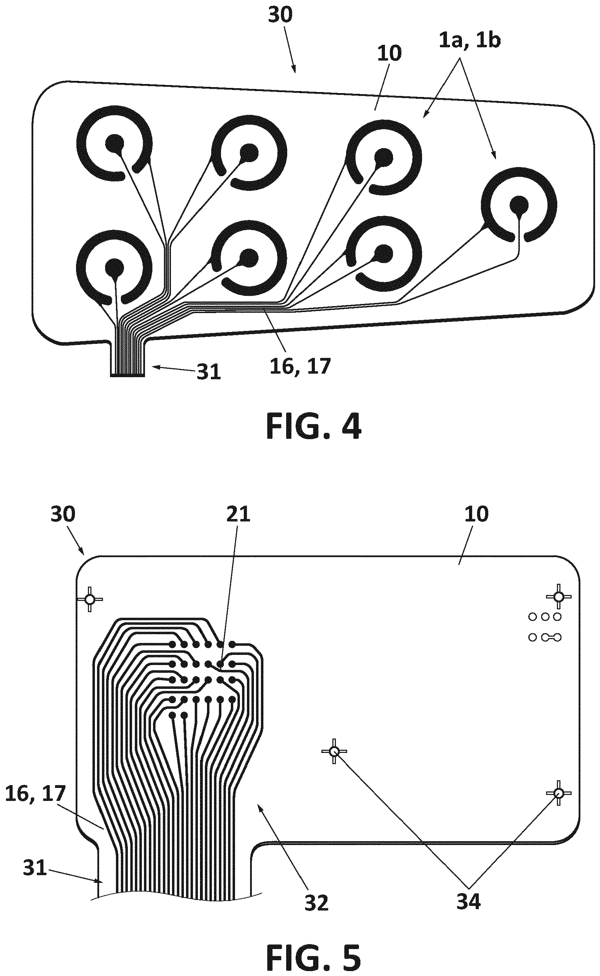

Figure 4 shows a first portion of multi-field electrode 30 with electrical stimulation electrodes in accordance with embodiments.

The stimulation electrodes illustrated are the electrodes 1a described with reference to Figures 1A-1D but it could be other electrodes as well, such as but without limitation, the electrodes 1b described with reference to Figure 2.

Preferably the entire multi-field electrode 30 is built on the same dielectric substrate 10, thus all the electrodes 1a are built on the same dielectric substrate 10 for an easier manipulation and production thereof. And to ease the manufacturing process, the same can occur with one or more of: the dielectric layer, the electrically conductive ink layer, the adhesive layer and the hydrogel layer.

The conductive tracks 16, 17 of the different electrodes 1a are preferably routed towards a connection harness 31 intended for electrical connection of the tracks 16, 17 to an apparatus for electrical stimulation. The connection harness 31 is preferably also formed on the dielectric substrate 10 and the tracks thereon are in the form of an electrically conductive layer like in the electrodes 1a, 1b, thereby forming a continuum that can be manufactured without requiring aligned attachment of different electrically conductive layers.

Figure 5 shows a second portion of a multi-field electrode 30 in accordance with embodiments.

The multi-field electrode 30 can be the same as that of Figure 4. The portion is at another end of the connection harness 31, and is also formed on the dielectric substrate 10. The routed conductive tracks 16, 17 end up in an area 32 with densely packed free ends 21 of the tracks 16, 17 for electrical connection with terminals of an apparatus for electrical stimulation.

In order to simplify the attachment of the multi-field electrode 30 to such an apparatus, the multi-field electrode 30 preferably includes attaching means 34, which in this case comprises holes formed on the dielectric substrate 10 that are intended to cooperate with attaching means of the apparatus like protrusions for instance.

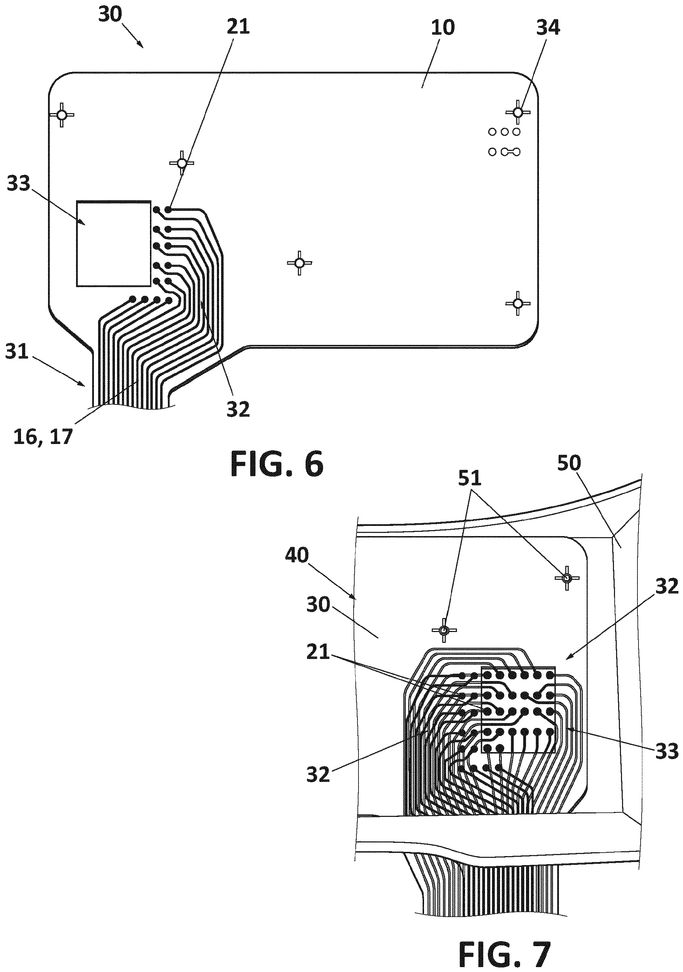

Figure 6 shows a portion of a multi-field electrode 30 in accordance with embodiments.

The multi-field electrode 30 can be part of a kit of multi-field electrodes. The portion is that for connecting the conductive tracks 16, 17 to an apparatus for electrical stimulation.

In contrast with the multi-field electrode 30 of Figure 5, the multi-field electrode of Figure 6 includes a cutout 33 in the dielectric substrate 10 intended to enable the connection of free ends 21 of other multi-field electrodes 30 with terminals of an apparatus for stimulation even with the presence of the multi-field electrode 30 in between.

Accordingly, the area 22 of other multi-field electrode(s) 30 that is to use the cutout 33 must be aligned with the cutout 33 so that the free ends 21 do not collide with the dielectric substrate 10 of the multi-field electrode 30 of Figure 6.

Figure 7 shows a portion of a kit 40 with of stimulation electrodes in accordance with embodiments. The portion illustrated is that intended for connection with an apparatus 50 for electrical stimulation, and is shown within part of a housing of such an apparatus 50.

The kit 40 includes two or more multi-field electrodes 30 like those of embodiments of Figures 4 to 6. In this embodiment, there are two multi-field electrodes 30, one of which includes a cutout 33 as described with reference to Figure 6. Accordingly, the free ends 21 of the different multi-field electrodes 30 can be connected with terminals of the apparatus 50 even if the areas 32 of the respective multi-field electrodes 30 are adjacent or one superposed to the other.

Further, in order to ease the arrangement of the multi-field electrodes 30 and the electrical connection of the free ends 21 to the terminals of the apparatus 50, the apparatus 50 includes attaching means 51, in this case in the form of protrusions, e.g. projecting mechanical elements on the housing. The attaching means 51 are preferably arranged such that they only accept one possible way of attachment of the multi-field electrodes 30, thereby making the areas 22 to be aligned with an area of the apparatus 50 with a set of terminals.

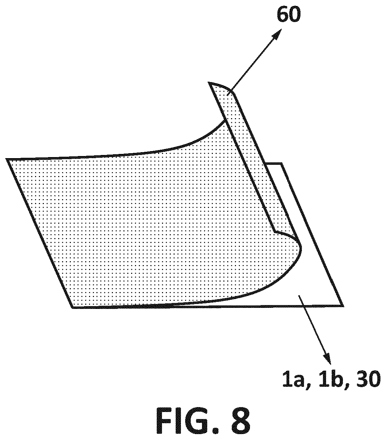

Figure 8 shows a stimulation electrode 1a, 1b or a multi-field electrode 30 in accordance with embodiments.

The stimulation electrode 1a, 1b or the multi-field electrode 30 may further comprise a protective layer 60 at least on top of the adhesive and/or hydrogel layers thereof to avoid accidental adhesion on some surface. Whenever the stimulation electrode 1a, 1b or the multi-field electrode 30 is to be attached to the body of the person, the protective layer 60 is peeled off so as to reveal the adhesive and hydrogel layers.

In this text, the terms first, second, etc. have been used herein to describe several devices, elements or parameters, it will be understood that the devices, elements or parameters should not be limited by these terms since the terms are only used to distinguish one device, element or parameter from another. For example, the first electrically conductive area could as well be named second electrically conductive area, and the second electrically conductive area could be named first electrically conductive area without departing from the scope of this disclosure.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A stimulation electrode (1a,1b) comprising:
a dielectric substrate (10);
an electrically conductive layer on the dielectric substrate (10), the electrically conductive layer comprising first and second electrically conductive areas (11a,11b,12a,12b), and first and second electrically conductive tracks (16,17) connected with the first and second areas (11a, 11b, 12a, 12b), respectively;
a dielectric layer (20) at least covering the conductive tracks (16,17); and
an adhesive layer (19) at least on a portion of the dielectric layer (20);
**characterized in that:**
the second electrically conductive area (12a,12b) encloses the first electrically conductive area (11a, 11b);
the second electrically conductive area (12a,12b) defining an opening (15) forming a passageway through which the first electrically conductive track (16) passes; and
the electrode further comprising an electrically conductive hydrogel layer (18) covering at least each of: the first electrically conductive area (11a, 11b), the second electrically conductive area (12a,12b) and the opening (15).

2. The stimulation electrode (1a,1b) of claim 1, wherein the first and second electrically conductive areas (11a, 11b,12a,12b) are concentric.

3. The stimulation electrode (1a, 1b) of any one of the preceding claims, wherein the first electrically conductive area (11a,11b) and the second electrically conductive area (12a,12b) have a perimeter with a same shape except for the opening (15) that the second electrically conductive area (12a, 12b) defines.

4. The stimulation electrode (1a, 1b) of any one of the preceding claims, wherein the second electrically conductive area (12a,12b) has an open geometry having two ends (12',12"), the opening (15) being formed in a space between the two ends (12', 12").

5. The stimulation electrode (1a,1b) of claim 4, wherein the two ends (12',12") are apart by a distance (D_{O}) equal to or less than 15% of a length of a longest side of a rectangular polygon (60) circumscribing the second electrically conductive area (12a,12b).

6. The stimulation electrode (1a, 1b) of any one of the preceding claims, wherein the second electrically conductive area (12a, 12b) encloses the first electrically conductive area (11a, 11b) with an angle (As) equal to or greater than 270°, preferably equal to or greater than 300° and more preferably equal to or greater than 330°.

7. A sheet (30) comprising a plurality of stimulation electrodes (1a, 1b) according to any one of the preceding claims such that the stimulation electrodes (1a, 1b) share the dielectric substrate.

8. A kit (40) of stimulation electrodes (1a, 1b), the kit comprising:
N sheets (30) according to the preceding claim, where N is a natural number equal to or greater than 2;
wherein each sheet (30) comprises an area (32) with a plurality of free ends (21) of the electrically conductive tracks (16,17) of the stimulation electrodes (1a,1b);
wherein for the second to the Nth sheet (30), being M each such sheet (30), the dielectric substrate (10) of the Mth sheet (30) has a cutout (33) that matches the area (32) with the plurality of free ends (21) of sheets (30) preceding the Mth sheet (30).

9. An apparatus (50) for electrical stimulation of a person, comprising:
at least one source for generating stimulation signals for electrical stimulation of the person;
a set of connection terminals for electrical connection of stimulation electrodes; and
one or more sheets (30) according to claim 7, or a kit (40) of stimulation electrodes (1a, 1b) according to claim 8, each stimulation electrode (1a, 1b) thereof being electrically connected with one or more connection terminals of the set of connection terminals.

10. The apparatus (50) of any one of the preceding claims, further comprising: user input means; and at least one processor configured to sequentially select, the location, the frequency and the intensity of the stimulation signals, in each terminal of the set of connection terminals, according to a stimulation technique, the stimulation technique being at least based on user feedback to stimuli applied to the person.

11. A method of manufacturing a stimulation electrode (1a, 1b), comprising:
arranging or manufacturing a dielectric substrate (10);
printing on the dielectric substrate (10), with electrically conductive ink, first and second electrically conductive areas (11a,11b,12a,12b), and first and second electrically conductive tracks (16,17) connected with the first and second electrically conductive areas (11a,11b,12a,12b), respectively;
adding a dielectric layer (20) at least covering the conductive tracks (16,17); and
applying an adhesive layer to the dielectric layer at least on a portion thereof not having the electrically conductive layer;
**characterized by:**
the second electrically conductive area (12a,12b) being printed such that it encloses the first electrically conductive area (11a,11b) printed;
the second electrically conductive area (12a,12b) being printed such that it defines an opening (15) forming a passageway through which the first electrically conductive track (16) passes; and
applying an electrically conductive hydrogel layer (18) to at least the first and second areas (11a, 11b, 12a, 12b) and the opening (15).

12. The method of claim 11, wherein the stimulation electrode (1a, 1b) is a stimulation electrode (1a, 1b) according to any one of claims 1-6.

## Patentansprüche

1. Eine Stimulationselektrode (1a, 1b) umfassend:
Ein dielektrisches Substrat (10);
eine elektrisch leitende Schicht auf dem dielektrischen Substrat (10), wobei die elektrisch leitende Schicht einen ersten und einen zweiten elektrisch leitenden Bereich (11a, 11b, 12a, 12b) und eine erste und eine zweite Leiterbahn (16, 17) umfasst, die mit dem ersten bzw. zweiten Bereich (11a, 11b, 12a, 12b) verbunden sind;
eine dielektrische Schicht (20), die zumindest die Leiterbahnen (16, 17) bedeckt; und eine Klebstoffschicht (19) zumindest auf einem Bereich der dielektrischen Schicht (20);
**dadurch gekennzeichnet, dass:**
Der zweite elektrisch leitende Bereich (12a, 12b) den ersten elektrisch leitenden Bereich (11a, 11b) umschließt;
der zweite elektrisch leitende Bereich (12a, 12b) eine Öffnung (15) definiert, die einen Durchgang bildet, durch den die erste Leiterbahn (16) verläuft;
und die Elektrode ferner eine elektrisch leitende Hydrogelschicht (18) umfasst, die mindestens jeden der folgenden Bereiche abdeckt: Den ersten elektrisch leitenden Bereich (11a, 11b), den zweiten elektrisch leitenden Bereich (12a, 12b) und die Öffnung (15).

2. Die Stimulationselektrode (1a, 1b) nach Anspruch 1, wobei der erste und der zweite elektrisch leitende Bereich (11a, 11b, 12a, 12b) konzentrisch sind.

3. Die Stimulationselektrode (1a, 1b) nach einem der vorhergehenden Ansprüche, wobei der erste elektrisch leitende Bereich (11a, 11b) und der zweite elektrisch leitende Bereich (12a, 12b) einen Umfang mit einer gleichen Form haben, mit Ausnahme der Öffnung (15), die der zweite elektrisch leitende Bereich (12a, 12b) definiert.

4. Die Stimulationselektrode (1a, 1b) nach einem der vorhergehenden Ansprüche, wobei der zweite elektrisch leitende Bereich (12a, 12b) eine offene Geometrie mit zwei Enden (12', 1") aufweist, wobei die Öffnung (15) in einem Raum zwischen den beiden Enden (12', 12") ausgebildet ist.

5. Die Stimulationselektrode (1a, 1b) nach Anspruch 4, wobei die beiden Enden (12', 12") um einen Abstand (Do) voneinander entfernt sind, der gleich oder weniger als 15% einer Länge einer längsten Seite eines rechteckigen Polygons (60) ist, das den zweiten elektrisch leitenden Bereich (12a, 12b) umschließt.

6. Die Stimulationselektrode (1a, 1b) nach einem der vorhergehenden Ansprüche, wobei der zweite elektrisch leitende Bereich (12a, 12b) den ersten elektrisch leitenden Bereich (11a, 11b) mit einem Winkel (As) gleich oder größer als 270°, bevorzugt gleich oder größer als 300° und noch bevorzugter gleich oder größer als 330° umschließt.

7. Eine Platte (30) umfassend eine Vielzahl von Stimulationselektroden (1a, 1b) nach einem der vorhergehenden Ansprüche, so dass die Stimulationselektroden (1a, 1b) das dielektrische Substrat gemeinsam nutzen.

8. Ein Kit (40) von Stimulationselektroden (1a, 1b), wobei der Kit folgendes umfasst:
N Platten (30) gemäß dem vorhergehenden Anspruch, wobei N eine natürliche Zahl gleich oder größer als 2 ist;
wobei jede Platte (30) einen Bereich (32) mit einer Vielzahl von freien Enden (21) der Leiterbahnen (16, 17) der Stimulationselektroden (1a, 1b) umfasst;
wobei für die zweite bis N-te Platte (30), wobei M jede derartige Platte (30) ist, bei der das dielektrische Substrat (10) der M-ten Platte (30) einen Ausschnitt (33) aufweist, der zu dem Bereich (32) mit der Vielzahl von freien Enden (21) der Platten (30) vor der M-ten Platte (30) passt.

9. Ein Apparat (50) zur elektrischen Stimulation einer Person, umfassend:
Mindestens eine Quelle zur Erzeugung von Stimulationssignalen für die elektrische Stimulation der Person;
einen Satz von Verbindungsanschlüssen für den elektrischen Anschluss von Stimulationselektroden;
und ein oder mehrere Platten (30) nach Anspruch 7 oder einen Kit (40) von Stimulationselektroden (1a, 1b) nach Anspruch 8, wobei jede Stimulationselektrode (1a, 1b) davon elektrisch mit einem oder mehreren Verbindungsanschlüssen des Satzes von Verbindungsanschlüssen verbunden ist.

10. Der Apparat (50) nach einem der vorangehenden Ansprüche, ferner umfassend:
Benutzereingabemittel; und mindestens einen Prozessor, der so konfiguriert ist, dass er sequentiell den Ort, die Frequenz und die Intensität der Stimulationssignale in jedem Anschluss des Satzes von Verbindungsanschlüssen gemäß einer Stimulationstechnik auswählt, wobei die Stimulationstechnik mindestens auf dem Benutzerfeedback zu den auf die Person ausgeübten Stimuli basiert.

11. Verfahren zur Herstellung einer Stimulationselektrode (1a, 1b), umfassend:
Anordnen oder Herstellen eines dielektrischen Substrats (10);
Bedrucken des dielektrischen Substrats (10) mit elektrisch leitender Tinte mit ersten und zweiten elektrisch leitenden Bereichen (11a, 11b, 12a, 12b) und ersten und zweiten Leiterbahnen (16, 17), die mit den ersten bzw. zweiten elektrisch leitenden Bereichen (11a, 11b, 12a, 12b) verbunden sind;
Hinzufügen einer dielektrischen Schicht (20), die zumindest die Leiterbahnen (16, 17) bedeckt;
und Aufbringen einer Klebstoffschicht auf die dielektrische Schicht zumindest in einem Bereich davon, der nicht die elektrisch leitende Schicht aufweist;
**gekennzeichnet durch**:
Der zweite elektrisch leitende Bereich (12a, 12b) so gedruckt wird, dass er den ersten gedruckten elektrisch leitenden Bereich (11a, 11b) umschließt;
der zweite elektrisch leitende Bereich (12a, 12b) so gedruckt wird, dass er eine Öffnung (15) definiert, die einen Durchgang bildet, durch den die erste Leiterbahn (16) verläuft;
und eine elektrisch leitende Hydrogelschicht (18) zumindest auf den ersten und zweiten Bereich (11a, 11b, 12a, 12b) und die Öffnung (15) aufgebracht wird.

12. Verfahren nach Anspruch 11, wobei die Stimulationselektrode (1a, 1b) eine Stimulationselektrode (1a, 1b) nach einem der Ansprüche 1-6 ist.

## Revendications

1. Électrode de stimulation (1a, 1b) comprenant :
un substrat diélectrique (10) ;
une couche électriquement conductrice sur le substrat diélectrique (10), la couche électriquement conductrice comprenant des première et deuxième zones (11a, 11b, 12a, 12b) électriquement conductrices, et des première et deuxième pistes (16, 17) électriquement conductrices connectées respectivement aux première et deuxième zones (11a, 11b, 12a, 12b) ;
une couche diélectrique (20) recouvrant au moins les pistes (16, 17) conductrices ; et
une couche adhésive (19) sur au moins une partie de la couche diélectrique (20) ;
**caractérisée en ce que** :
la deuxième zone (12a, 12b) électriquement conductrice renferme la première zone (11a, 11b) électriquement conductrice ;
la deuxième zone (12a, 12b) électriquement conductrice définit une ouverture (15) formant un passage à travers lequel passe la première piste (16) électriquement conductrice ; et
l'électrode comprend en outre une couche d'hydrogel (18) électriquement conductrice recouvrant au moins chacune parmi : la première zone (11a, 11b) électriquement conductrice, la deuxième zone (12a, 12b) électriquement conductrice et l'ouverture (15).

2. Électrode de stimulation (1a, 1b) selon la revendication 1, dans laquelle les première et deuxième zones (11a, 11b, 12a, 12b) électriquement conductrices sont concentriques.

3. Électrode de stimulation (1a, 1b) selon l'une des revendications précédentes, dans laquelle la première zone (11a, 11b) électriquement conductrice et la deuxième zone (12a, 12b) électriquement conductrice ont un périmètre de même forme à l'exception de l'ouverture (15) définie par la deuxième zone (12a, 12b) électriquement conductrice.

4. Électrode de stimulation (1a, 1b) selon l'une des revendications précédentes, dans laquelle la deuxième zone (12a, 12b) électriquement conductrice a une géométrie ouverte ayant deux extrémités (12', 12"), l'ouverture (15) étant formée dans un espace entre les deux extrémités (12', 12").

5. Électrode de stimulation (1a, 1b) selon la revendication 4, dans laquelle les deux extrémités (12', 12") sont séparées par une distance (Do) égale ou inférieure à 15 % d'une longueur du côté le plus long d'un polygone rectangulaire (60) circonscrivant la deuxième zone (12a, 12b) électriquement conductrice.

6. Électrode de stimulation (1a, 1b) selon l'une des revendications précédentes, dans laquelle la deuxième zone (12a, 12b) électriquement conductrice renferme la première zone (11a, 11b) électriquement conductrice avec un angle (As) égal ou supérieur à 270°, de préférence égal ou supérieur à 300° et plus préférentiellement égal ou supérieur à 330°.

7. Feuille (30) comprenant une pluralité d'électrodes de stimulation (1a, 1b) selon l'une des revendications précédentes telles que les électrodes de stimulation (1a, 1b) partagent le substrat diélectrique.

8. Ensemble (40) d'électrodes de stimulation (1a, 1b), l'ensemble comprenant :
N feuilles (30) selon la revendication précédente, où N est un nombre naturel égal ou supérieur à 2 ;
dans lequel chaque feuille (30) comprend une zone (32) avec une pluralité d'extrémités libres (21) des pistes (16, 17) électriquement conductrices des électrodes de stimulation (1a, 1b) ;
dans lequel pour les deuxième à N^{ième} feuilles (30) étant M chacune de ces feuilles (30), le substrat diélectrique (10) de la M^{ième} feuille (30) a une découpe (33) qui correspond à la zone (32) avec la pluralité d'extrémités libres (21) des feuilles (30) précédant la M^{ième} feuille (30).

9. Appareil (50) pour la stimulation électrique d'une personne, comprenant :
au moins une source pour générer des signaux de stimulation pour la stimulation électrique de la personne ;
un ensemble de bornes de connexion pour la connexion électrique d'électrodes de stimulation ; et
une ou plusieurs feuilles (30) selon la revendication 7, ou un ensemble (40) d'électrodes de stimulation (1a, 1b) selon la revendication 8, chaque électrode de stimulation (1a, 1b) de ce dernier étant connectée électriquement à une ou plusieurs bornes de connexion de l'ensemble de bornes de connexion.

10. Appareil (50) selon l'une quelconque des revendications précédentes, comprenant en outre : des moyens d'entrée de l'utilisateur ; et au moins un processeur configuré pour sélectionner séquentiellement l'emplacement, la fréquence et l'intensité des signaux de stimulation dans chaque borne de l'ensemble de bornes de connexion selon une technique de stimulation, la technique de stimulation étant au moins basée sur les réactions de l'utilisateur aux stimuli appliqués à la personne.

11. Méthode de fabrication d'une électrode de stimulation (1a, 1b), comprenant :
l'agencement ou la fabrication d'un substrat diélectrique (10) ;
à l'aide d'une encre électriquement conductrice, l'impression sur le substrat diélectrique (10) de première et deuxième zones (11a, 11b, 12a, 12b) électriquement conductrices, et de première et deuxième pistes (16, 17) électriquement conductrices connectées respectivement aux première et deuxième zones (11a, 11b, 12a, 12b) électriquement conductrices ;
l'ajout d'une couche diélectrique (20) recouvrant au moins les pistes (16, 17) conductrices ; et
l'application d'une couche adhésive sur la couche diélectrique sur au moins une partie de celle-ci ne comportant pas de couche électriquement conductrice ;
**caractérisée par** :
la deuxième zone (12a, 12b) électriquement conductrice imprimée de telle sorte qu'elle renferme la première zone (11a, 11b) électriquement conductrice imprimée ;
la deuxième zone (12a, 12b) électriquement conductrice étant imprimée de manière à définir une ouverture (15) formant un passage à travers lequel passe la première piste (16) électriquement conductrice ; et
l'application d'une couche d'hydrogel électriquement conductrice (18) sur au moins les première et deuxième zones (11a, 11b, 12a, 12b) et l'ouverture (15).

12. Méthode selon la revendication 11, dans laquelle l'électrode de stimulation (1a, 1b) est une électrode de stimulation (1a, 1b) selon l'une des revendications 1 à 6.
